# EUROPÄISCHE PATENTSCHRIFT

(11) **EP 0 546 396 B1**
(45) Veröffentlichungstag und Bekanntmachung des Hinweises auf die Patenterteilung: **28.02.1996**
(21) Anmeldenummer: 92120259.4
(22) Anmeldetag: 27.11.1992
(51) Int. Cl.: C07D 309/06

(54) **Verfahren zur Herstellung von 4-Hydroxymethyl-tetrahydropyranen**
Method for the preparation of 4-hydroxymethyltetrahydropyrans
Méthode pour la préparation des 4-hydroxyméthyltétrahydropyranes

(30) Priorität: 13.12.1991 DE 4141222
(43) Veröffentlichungstag der Anmeldung: 16.06.1993
(73) Patentinhaber: BASF Aktiengesellschaft, D-67063 Ludwigshafen (DE)
(72) Erfinder: Henkelmann, Jochem, Dr., W-6700 Ludwigshafen (DE); Ruehl, Thomas, Dr., W-6710 Frankenthal (DE); Zimmermann, Horst, Dr., W-6800 Mannheim 24 (DE); Goetz, Norbert, Dr., W-6520 Worms 1 (DE); Spiegler, Wolfgang, Dr., W-6520 Worms 27 (DE); Kuekenhoehnen, Thomas, Dr., W-6737 Boehl-Iggelheim (DE)

(56) Entgegenhaltungen:
- JOURNAL OF THE AMERICAN CHEMICAL SOCIETY, Bd. 98, Nr. 20, 29. September 1976, Washington, DC, US, Seiten 6350 - 6353 W.H. RASTETTER: 'sym-Oxepin oxide'
- CHEMICAL ABSTRACTS, vol. 105, no. 17, 27. Oktober 1986, Columbus, Ohio, US; abstract no. 152925e, Seite 682

## Beschreibung

Die vorliegende Erfindung betrifft ein Verfahren zur Herstellung von 4-Hydroxymethyl-tetrahydropyranen durch katalytische Hydrierung von Tetrahydropyran-4-carbonsäureestern.

Die Synthese von 4-Hydroxymethyl-tetrahydropyran ausgehend von 4-Methylentetrahydropyran durch Epoxidierung mit Wasserstoffperoxid und Reduktion des Epoxids ist aus SU-A-84-3722686 bekannt. Dabei bereitet die Darstellung der Ausgangsverbindung Schwierigkeiten durch Doppelbindungs-Isomerisierung.

Weiterhin läßt sich 4-Hydroxymethyl-tetrahydropyran aus dem entsprechenden Aldehyd durch Reduktion darstellen (J. Am. Chem. Soc., 98, 6350 bis 6353 (1976)). Auch hier stellt die Darstellung des Aldehyds eine vielstufige Synthese dar.

Es bestand daher die Aufgabe, den zuvor genannten Nachteilen abzuhelfen, und ein Verfahren zu entwickeln, das von Tetrahydropyran-4-carbonsäureestern direkt zu 4-Hydroxymethyl-tetrahydropyranen führt.

Demgemäß wurde ein neues und verbessertes Verfahren zur Herstellung von 4-Hydroxymethyl-tetrahydropyranen der allgemeinen Formel I 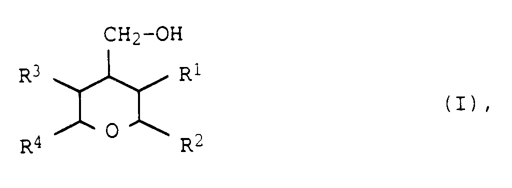
in der
- R¹,R²,R³,R⁴: Wasserstoff, C₁- bis C₁₂-Alkyl, C₃- bis C₈-Cycloalkyl, Aryl oder C₇- bis C₂₀-Aralkyl
bedeuten, gefunden, welches dadurch gekennzeichnet ist, daß man Tetrahydropyran-carbonsäureester der allgemeinen Formel II 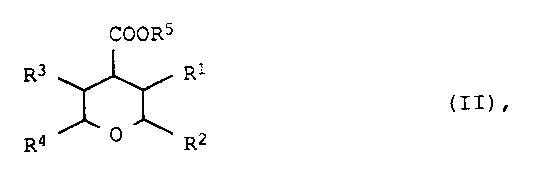
in der R¹, R², R³ und R⁴ die obengenannten Bedeutungen hat und R⁵ Wasserstoff und C₁- bis C₁₂-Alkyl bedeutet, bei Temperaturen von 50 bis 400°C und Drücken von 1 bis 400 bar in Gegenwart von Wasserstoff und Hydrierkatalysatoren umsetzt.

Das erfindungsgemäße Verfahren läßt sich wie folgt durchführen:
Die Tetrahydropyran-4-carbonsäureester der allgemeinen Formel II können bei erhöhter Temperatur in Gegenwart von Hydrierkatalysatoren mit Wasserstoff in Hydroxymethylverbindungen der allgemeinen Formel I umgewandelt werden.

Die Umsetzung kann man diskontinuierlich oder kontinuierlich bei Temperaturen von 50 bis 400°C, vorzugsweise von 150 bis 350°C, insbesondere von 180 bis 260°C durchführen. Der Druck bei der Reaktion beträgt 1 bis 400 bar, insbesondere 50 bis 300 bar.

Die Umsetzung der Tetrahydropyran-4-carbonsäureester II zu Hydroxymethylverbindungen I kann in der Gasphase bei Temperaturen von 50 bis 400°C und Drücken von 1 bis 400 bar oder bevorzugt in der Flüssigphase bei Temperaturen von 50 bis 400°C und Drücken von 1 bis 400 bar erfolgen. Man arbeitet dabei vorteilhaft mit einer Katalysatorbelastung von 0,01 bis 5 g, bevorzugt 0,2 bis 1 g, besonders bevorzugt 0,05 bis 0,5 g Tetra hydropyran-4-carbonsäureester II pro g Katalysator und Stunde.

Alle Einsatzstoffe können in fester, flüssiger oder gasförmiger Form in das erfindungsgemäße Verfahren eingebracht werden.

Die Reaktion kann in der Sumpf- oder Rieselfahrweise durchgeführt werden, wobei der Hydrierkatalysator in der Regel fest angeordnet wird. Es ist auch möglich, suspendierte Katalysatoren einzusetzen. Als Reaktoren können z.B. Rohrreaktoren oder Rohrbündelreaktoren verwendet werden.

Die erfindungsgemäße Umsetzung kann in Abwesenheit von Lösungsmitteln durchgeführt werden. Es kann jedoch vorteilhaft sein, in Anwesenheit von Lösungsmitteln zu arbeiten. Als Lösungsmittel können beispielsweise Wasser, Ether wie Diethylether, Tetrahydrofuran und Dioxan, Aromaten wie Benzol, Toluol und die Xylole, chlorierte Kohlenwasserstoffe wie Chloroform und Methylenchlorid, Alkohole, z.B. C₁- bis C₈-Alkanole, bevorzugt C₁- bis C₅-Alkanole wie Methanol, Ethanol, n-Propanol, iso-Propanol, die Butanole und die Pentanole oder Gemische dieser Lösungsmittel verwendet werden. Als besonders vorteilhaft haben sich Alkohole erwiesen. Die Menge an Lösungsmittel kann in weiten Grenzen variiert werden und beträgt bevorzugt, bezogen auf den eingesetzten Tetrahydropyran-4-carbonsäureester II, 5 bis 90 Gew.-%; besonders bevorzugt ist die Hydrierung ohne Lösungsmittel.

Die Hydrierkatalysatoren können allgemein übliche Katalysatoren sein, wie sie z.B. in H. Kropf, Methoden der organischen Chemie (Houben-Weyl), Band IV/1c, Georg Thieme Verlag Stuttgart, 1980, beschrieben sind.

Bevorzugte Hydrierkatalysatoren sind solche, die Kupfer und/oder einen oder mehrere Metalle der VII. und/oder VIII. Nebengruppe des Periodischen Systems der Elemente enthalten, insbesondere Nickel, Cobalt, Palladium, Rhodium, Platin, Ruthenium, Rhenium. Sie können sowohl als Trägerkatalysatoren oder in kompakter Form, d.h. ohne Träger eingesetzt werden. Als Trägermaterial können Siliciumdioxid, Aluminiumoxide, Titanoxide, Aktivkohle, Silikate oder Zeolithe verwendet werden.

Die Umsetzung der Tetrahydropyran-4-carbonsäureester II in der Flüssigphase wird beispielsweise so durchgeführt, daß man ein Gemisch aus II und gegebenenfalls einem Lösungsmittel in Gegenwart eines suspendierten Hydrierkatalysators und Wasserstoff unter Druck auf die gewünschte Reaktionstemperatur erhitzt. Nach Ablauf der Reaktion wird das Reaktionsgemisch abgekühlt und entspannt und der Katalysator, z.B. durch Filtration entfernt. Das Reaktionsgemisch kann anschließend zur Gewinnung der gewünschten Hydroxymethylverbindung I z.B. fraktionierend destilliert werden.

Arbeitet man, was besonders bevorzugt ist, in Gegenwart eines als Festbett angeordneten Hydrierkatalysators in der Flüssigphase, so leitet man den Tetrahydropyran-4-carbonsäureester II, gegebenenfalls zusammen mit einem Lösungsmittel, in Gegenwart von Wasserstoff unter Druck in Sumpf- oder Rieselfahrweise über den Hydrierkatalysator. Der Hydrieraustrag wird abgekühlt und entspannt und anschließend zur Gewinnung der Hydroxymethylverbindung I z.B. fraktionierend destilliert.

Die Substituenten R¹, R², R³, R⁴ und R⁵ in den Verbindungen I und II haben folgende Bedeutungen:
R¹, R², R³, R⁴
- unabhängig voneinander
- Wasserstoff,
- C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Undecyl, n-Dedecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
R⁵
- Wasserstoff,
- C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl.

Die für die erfindungsgemäße Umsetzung benötigten Tetrahydropyran-4-carbonsäureester II sind ausgehend von Tetrahydropyran-4,4-dicarbonsäurediethylester (J. Chem. Soc. Seite 2268 (1952)) zugänglich. Der Diethylether kann durch Verseifung mit Kaliumhydroxid zu der Tetrahydropyran-4,4-dicarbonsäure, anschließender thermischer Decarboxylierung zur Tetrahydropyran-4-carbonsäure, nachfolgender Reaktion mit Thionylchlorid zu Tetrahydropyran-4-carbonsäurechlorid und anschließender Veresterung mit Alkoholen zu den Tetrahydropyran-4-carbonsäureestern der Formel II umgewandelt werden (J. Chem. Soc. S. 2525 (1930)).

Die erfindunsgemäß herstellbaren 4-Hydroximethyl-tetrahydropyrane sind vor allem als Vorprodukt zur der Herstellung von Pflanzenschutzmitteln geeignet.

### Beispiele

Die in den Beispielen verwendeten Katalysatoren waren wie folgt zusammengesetzt (Angaben in Gew.-%):

| Katalysator | Zusammensetzung |
|---|---|
| A | 64 % CoO; 18 % CuO; 7 % MnO₂; 4 % MoO₂; 0,2 % Na₂O |
| B | 50 % NiO; 30 % ZrO₂; 18 % CuO; 1,5 % MoO₂; 0,2 % Na₂O |
| C | 35 % CuO; 65 % Al₂O₃ |
| D | 52 % CuO; 48 % Al₂O₃ |
| E | 50 % Cu; 48 % Cr; 2 % MnO₂ |
| F | 3 % Pd; 3 % Re; 94 % TiO₂ |
| G | 1 % Ru; 1,2 % Sn; 1,3 % B; 96,5 % Al₂O₃ |

### Beispiel 1

Die hydrierende Umsetzung wurde in einem 300-ml-Rührautoklav durchge führt, in dem sich 10 g des Katalysators A und 100 ml Tetrahydropyran-4-carbonsäuremethylester befanden. Die Reaktionsbedingungen waren 250°C/260 bar Wasserstoffdruck während 12 h.

Die gaschromatographische Untersuchung des flüssigen Reaktionsaustrages ergab eine Ausbeute von 97 %.

### Beispiel 2 bis 7

Die hydrierenden Umsetzungen wurden analog zu Beispiel 1 durchgeführt. Die jeweils verwendeten Katalysatoren sind zusammen mit der Zusammensetzung des Reaktionsaustrages an Tetrahydropyran-4-carbonsäuremethylester und 4-Hydroxymethyl-tetrahydropyran in Tabelle 1 wiedergegeben.

**Tabelle 1**

| Katalysator | Tetrahydropyran-4-carbonsäuremethylester | 4-Hydroxymethyltetrahydropyran |
|---|---|---|
| | [GC-Fl.-%] | [GC-Fl.-%] |
| B | 0,8 | 97 |
| C | 0,2 | 96 |
| D | 1,5 | 90 |
| E | 1,5 | 94 |
| F | 2,5 | 81 |
| G | 0,3 | 94 |

### Beispiel 8 bis 11

Die Umsetzungen wurden analog zu Beispiel 1 in einem 2-l-Rührautoklav mit 1 l Tetrahydropyran-4-carbonsäuremethylester und 50 g Kontakt durchgeführt. Die Reaktionsbedingungen waren 250°C/300 bar Wasserstoffdruck während 60 h.

Die jeweils verwendeten Katalysatoren sind zusammen mit der Zusammensetzung des Hydrieraustrages in Tabelle 2 dargestellt.

**Tabelle 2**

| Kontakt | Tetrahydropyran-4-carbonsäuremethylester | 4-Hydroxymethyltetrahydropyran |
|---|---|---|
| | [GC-Fl.-%] | [GC-Fl.-%] |
| A | 2,5 | 92 |
| C | 0,4 | 96 |
| D | - | 72 |
| G | 0,7 | 87 |

### Beispiel 12

Die hydrierende Umsetzung wurde in einem Rohrreaktor (Länge 2000 mm; Durchmesser: 16 mm), in dem sich der Kontakt C in einem Festbett befand, durchgeführt. Der Reaktor wurde über einen außenliegenden Heizmantel mit Öl auf die Reaktionstemperatur aufgeheizt. Die gasförmigen und flüssigen Einsatzstoffe durchströmten den Reaktor von oben nach unten (Rieselfahrweise). Der Hydrieraustrag wurde entspannt und in einem Gas-Flüssigkeits-Abscheider in seine gasförmigen und flüssigen Bestandteile zerlegt.

Der Katalysator wurde in Form von 2,5 bis 4 mm großem Splitt eingesetzt und vor Beginn der Hydrierung mit Wasserstoff aktiviert.

Bei einem Gesamtdruck von 300 bar und einer Temperatur von 250°C wurden dem Reaktor stündlich 0,3 kg Tetrahydropyran-4-carbonsäuremethylester je Liter Katalysator sowie 0,5 m³ Wasserstoff je Liter Katalysator zugeführt.

Die gaschromatographische Untersuchung des flüssigen Reaktionsaustrages ergab bei vollständigem Umsatz eine Selektivität von 97 %.

## Patentansprüche

1. Verfahren zur Herstellung von 4-Hydroxymethyl-tetrahydropyranen der allgemeinen Formel I 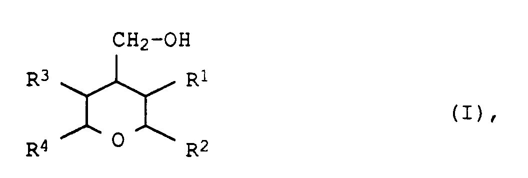 in der
R¹,R²,R³,R⁴ Wasserstoff, C₁- bis C₁₂-Alkyl, C₃- bis C₈-Cycloalkyl, Aryl oder C₇- bis C₂₀-Aralkyl
bedeuten, dadurch gekennzeichnet, daß man Tetrahydropyran-carbonsäureester der allgemeinen Formel II 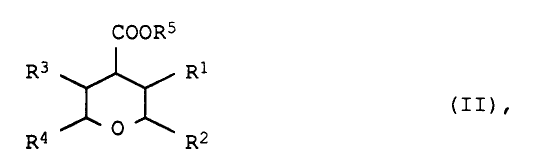 in der R¹, R², R³ und R⁴ die obengenannten Bedeutungen hat und R⁵ Wasserstoff und C₁- bis C₁₂-Alkyl bedeutet, bei Temperaturen von 50 bis 400°C und Drücken von 1 bis 400 bar in Gegenwart von Wasserstoff und Hydrierkatalysatoren umsetzt.

2. Verfahren nach Anspruch 1, dadurch gekennzeichnet, daß man Hydrierkatalysatoren verwendet, die Kupfer und/oder ein oder mehrere Metalle der Elemente der VII. und/oder VIII. Nebengruppe des Periodensystems der Elemente enthalten.

3. Verfahren nach Anspruch 1, dadurch gekennzeichnet, daß man in der Flüssigphase arbeitet.

## Claims

1. A process for the preparation of 4-hydroxymethyltetrahydropyrans of the formula I 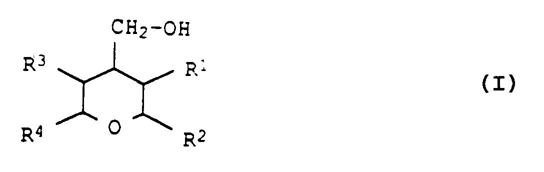 where
R¹, R², R³ and R⁴ are hydrogen, C₁- to C₁₂-alkyl, C₃- to C₈-cycloalkyl, aryl or C₇- to C₂₀-aralkyl,
which comprises reacting a tetrahydropyrancarboxylic ester of the formula II 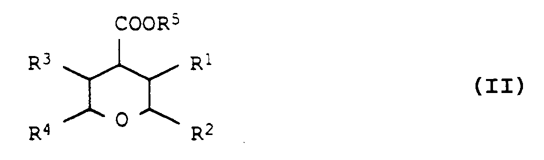 where R¹, R², R³ and R⁴ are as defined above, and R⁵ is hydrogen or C₁- to C₁₂-alkyl,
at from 50 to 400°C and at from 1 to 400 bar in the presence of hydrogen and a hydrogenation catalyst.

2. A process as claimed in claim 1, wherein the hydrogenation catalyst used contains copper and/or one or more metals of elements from sub-group VII and/or VIII of the Periodic Table of the Elements.

3. A process as claimed in claim 1, which is carried out in a liquid phase.

## Revendications

1. Procédé de préparation de 4-hydroxyméthyltetrahydropyrannes de formule générale I 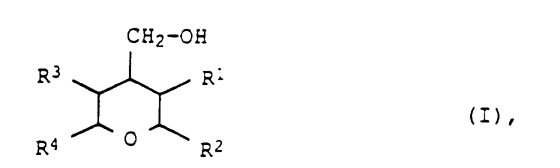 dans laquelle
R¹, R², R³, R⁴ représentent des atomes d'hydrogène ou des restes alkyle en C₁-C₁₂, cycloalkyle en C₃-C₈, aryle ou aralkyle en C₇₋C₂₀,
caractérisé en ce que l'on fait réagir des esters d'acide tétrahydropyranne-carboxylique de formule générale II 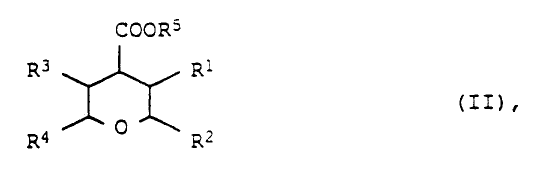 dans laquelle R¹, R², R³ et R⁴ ont les mêmes significations que précédemment et R⁵ représente un atome d'hydrogène ou un reste alkyle en C₁₋C₁₂, a des températures de 50 à 400°C et sous des pressions de 1 à 400 bar, en présence d'hydrogène et de catalyseurs d'hydrogénation.

2. Procédé selon la revendication 1, caractérisé en ce que l'on utilise des catalyseurs d'hydrogénation qui contiennent du cuivre et/ou un ou plusieurs métaux des groupes VIIB et/ou VIII de la classification périodique des éléments.

3. Procédé selon la revendication 1, caractérisé en ce que l'on procéde en phase liquide.
